# EUROPEAN PATENT APPLICATION

(11) **EP 3 919 100 A1**
(43) Date of publication of application: **08.12.2021**
(21) Application number: 20776404.4
(22) Date of filing: 19.02.2020
(51) Int. Cl.: A61M 5/158, A61M 25/06

(54) **INDWELLING SUBCUTANEOUS ADMINISTRATION NEEDLE**

(30) Priority: 27.03.2019 JP 2019060414
(71) Applicant: TERUMO Kabushiki Kaisha, Tokyo 151-0072 (JP)
(72) Inventor: MAEKAWA, Minami, Ashigarakami-gun, Kanagawa 259-0151 (JP); ARINOBE, Manabu, Ashigarakami-gun, Kanagawa 259-0151 (JP); IWASE, Yoichiro, Ashigarakami-gun, Kanagawa 259-0151 (JP)
(74) Representative: Casalonga
(86) International application number: PCT/JP2020/006456
(87) International publication number: WO 2020/195364

(57) **Abstract**

Provided is an indwelling needle for subcutaneous administration (10) indwelled in subcutaneous tissue for subcutaneously administering a medicinal solution, the indwelling needle for subcutaneous administration (10) provided with an inner needle (12) including an inner needle main body (14) at a distal end of which a sharp needle tip (26) is formed, and an inner needle hub (16) provided on a proximal end side of the inner needle main body (14), and an outer needle (22) including an outer needle main body (18) including a lumen (30) through which the inner needle (12) is inserted, and an outer needle hub (20) provided on a proximal end side of the outer needle main body (18) in which a hub insertion portion (32) in which the inner needle hub (16) is mounted is formed, in which the needle tip (26) of the inner needle (12) protrudes by 2.5 to 9 mm in an axis direction from a distal end (28) of the outer needle (22) in a state in which the inner needle hub (16) is mounted in the outer needle hub (20) .

## Description

### Technical Field

The present invention relates to an indwelling needle for subcutaneous administration indwelled in a living body for administering a medicinal solution.

### Background Art

In a case of administering medicine into a living body, an indwelling needle (catheter assembly) having a double structure formed of an inner needle on which a sharp needle tip is formed and a tubular outer needle (catheter) formed so as to cover the inner needle is used. A distal end of the outer needle is formed obtusely, and this is introduced into the living body in a state in which the sharp inner needle protrudes from the distal end of the outer needle. After the introduction into the living body, the inner needle is pulled out, so that the outer needle is indwelled in the living body. Then, a medicinal solution is administered into the living body through a lumen of the outer needle. The indwelling needle mainly punctures a blood vessel (vein and the like) to be used, but is indwelled in subcutaneous tissue in some cases.

For example, JP 2016-127872 A relates to an indwelling needle indwelled subcutaneously in order to administer a medicinal solution subcutaneously, and discloses the indwelling needle in which a puncture portion has a double structure of an inner needle and an outer needle.

### Summary of Invention

However, in a case where an outer needle is indwelled in subcutaneous tissue, there is a problem that the outer needle is easily broken or blocked. That is, there is a case where the outer needle advances to a distal end side due to vibration when the outer needle is fixed to skin, or a case where the outer needle advances due to movement of a living body. In such a case, the outer needle might hit the subcutaneous tissue to cause breakage (kink). There is a risk that a distal end side of the outer needle is blocked by wall-shaped subcutaneous tissue, and administration of a medicinal solution through a lumen is delayed, so that an administration pressure increases.

In a case of intermittently administering the medicinal solution at intervals of about few hours while keeping the indwelling needle indwelled in the living body, protein, cells and the like might gather in the lumen of the outer needle to generate a thrombotic mass while the medicinal solution does not flow. In such a case, there is a problem that the thrombus cannot be discharged from the lumen of the outer needle, and the indwelling needle is blocked.

Therefore, there is a need for an indwelling needle for subcutaneous administration capable of preventing kink and blockage even in a case of indwelling in the subcutaneous tissue.

One aspect of the following disclosure is an indwelling needle for subcutaneous administration indwelled in subcutaneous tissue for subcutaneously administering a medicinal solution, the indwelling needle for subcutaneous administration provided with an inner needle including an inner needle main body at a distal end of which a sharp needle tip is formed, and an inner needle hub provided on a proximal end side of the inner needle main body, and an outer needle including an outer needle main body including a lumen through which the inner needle is inserted, and an outer needle hub provided on a proximal end side of the outer needle main body in which a hub insertion portion in which the inner needle hub is mounted is formed, in which the needle tip of the inner needle protrudes by 2.5 to 9 mm in an axis direction from a distal end of the outer needle in a state in which the inner needle hub is mounted in the outer needle hub.

According to the indwelling needle for subcutaneous administration in the above-described aspect, the kink and blockage may be prevented even in a case of indwelling in the subcutaneous tissue.

### Brief Description of Drawings

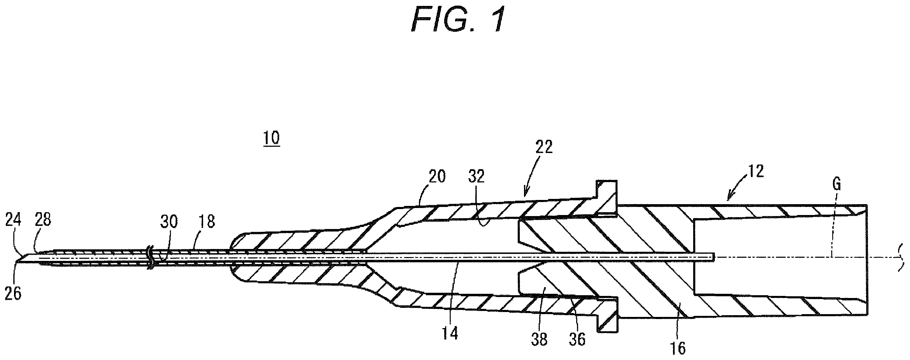

Fig. 1 is a cross-sectional view of an indwelling needle for subcutaneous administration according to a first embodiment.

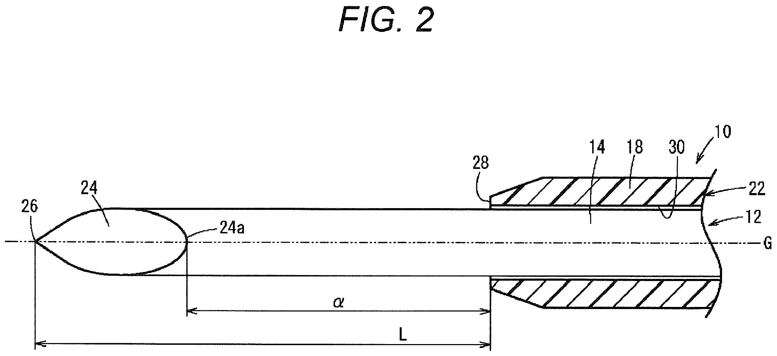

Fig. 2 is an enlarged view of the vicinity of a distal end of the indwelling needle for subcutaneous administration in Fig. 1.

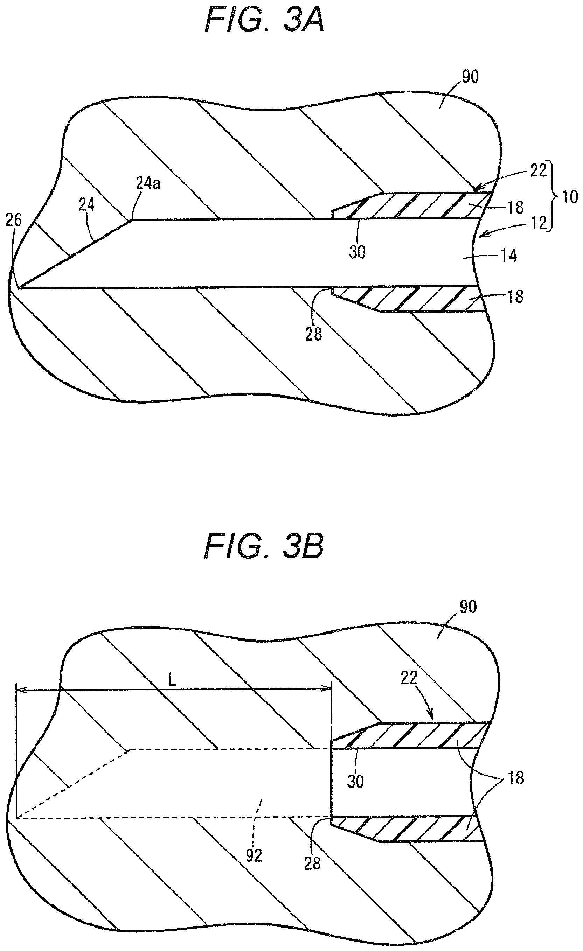

Fig. 3A is an explanatory view illustrating a state in which the indwelling needle for subcutaneous administration in Fig. 1 punctures subcutaneous tissue, and Fig. 3B is an explanatory view illustrating a state in which an inner needle is pulled out of the indwelling needle for subcutaneous administration in Fig. 1.

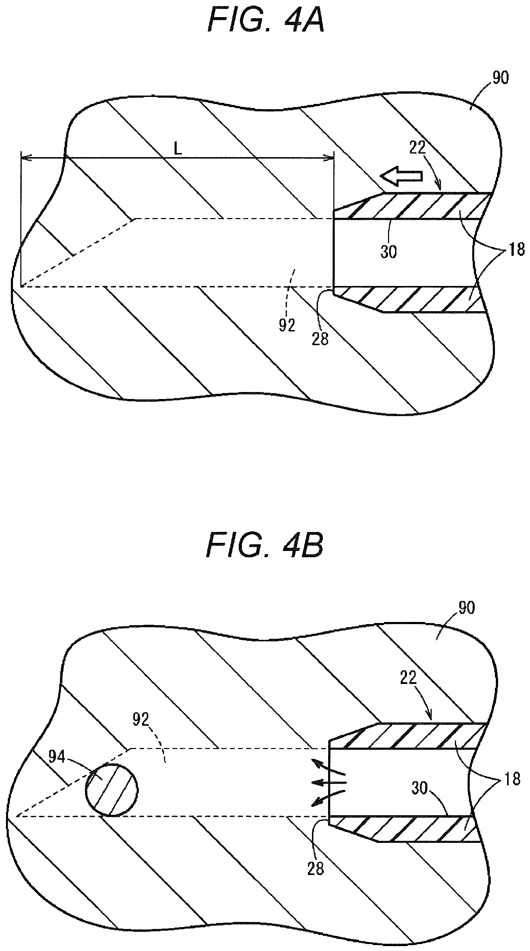

Fig. 4A is an explanatory view illustrating a state in which an outer needle of the indwelling needle for subcutaneous administration in Fig. 1 is displaced to a distal end side, and Fig. 4B is an explanatory view illustrating a state in which a precipitate is discharged from the outer needle of the indwelling needle for subcutaneous administration in Fig. 1.

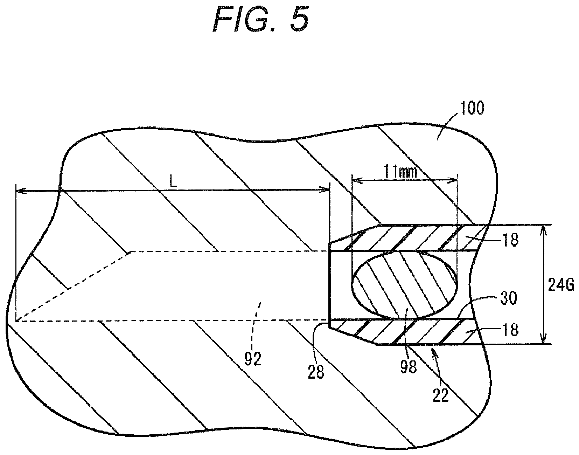

Fig. 5 is an explanatory view of an experimental example of the first embodiment, illustrating an example in which porcine subcutaneous tissue is punctured with an indwelling needle for subcutaneous administration of 24 G and a simulated thrombus is discharged.

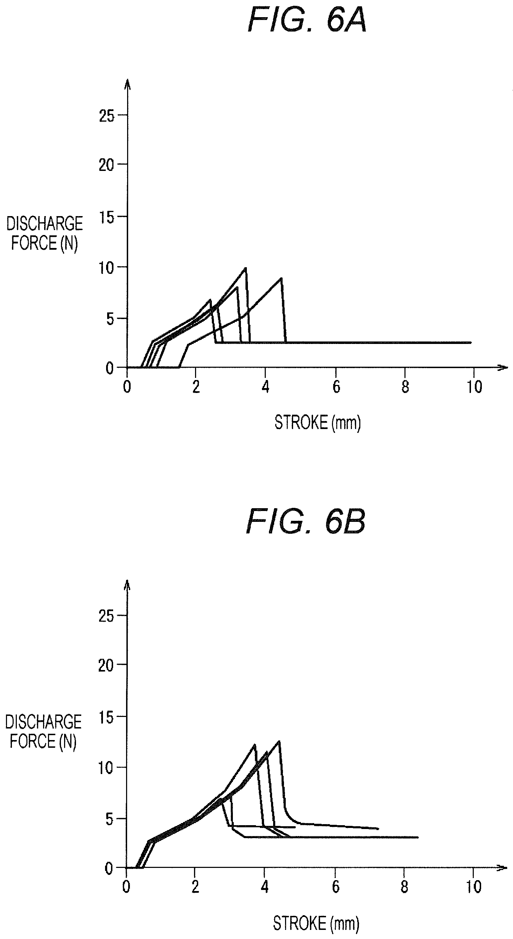

Fig. 6A is a graph illustrating a pressure required for discharging the simulated thrombus in the indwelling needle for subcutaneous administration of Reference Example 1 in a state in which this does not puncture the subcutaneous tissue, and Fig. 6B is a graph illustrating a pressure required for discharging the simulated thrombus in a case where an inner needle is protruded from an outer needle by 4 mm in the indwelling needle for subcutaneous administration according to Example 1.

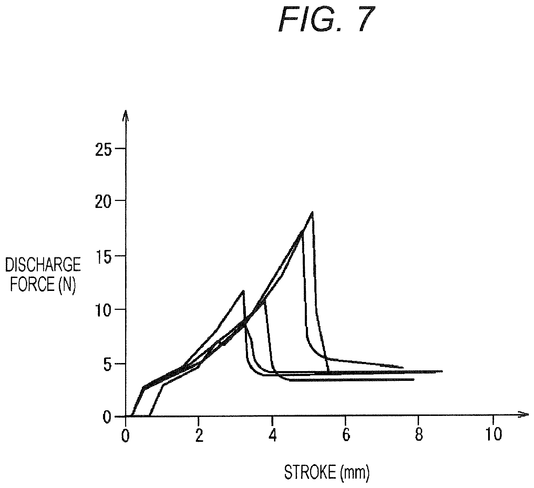

Fig. 7 is a graph illustrating a pressure required for discharging the simulated thrombus in a case where the inner needle is not protruded from the outer needle in the indwelling needle for subcutaneous administration according to Comparative Example 1.

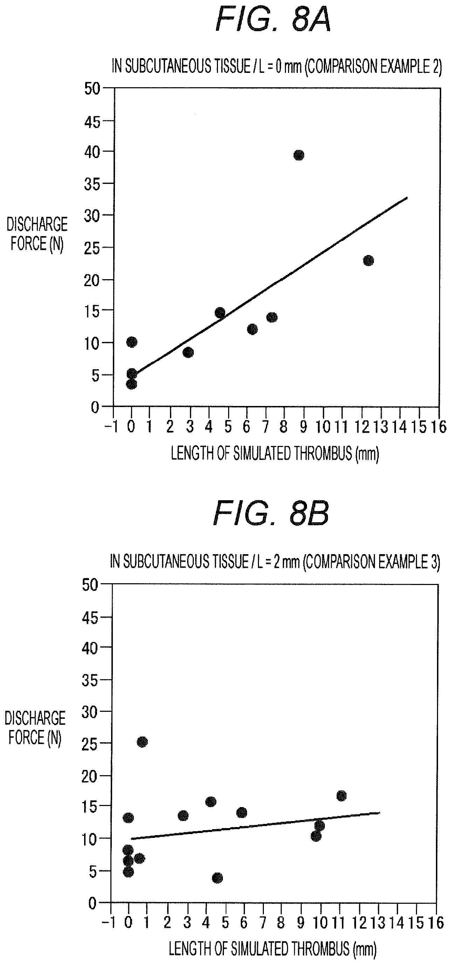

Fig. 8A is a graph illustrating a measurement result of a length of the simulated thrombus and an administration pressure in Comparative Example 2, and Fig. 8B is a graph illustrating a measurement result of a length of the simulated thrombus and an administration pressure in Comparative Example 3.

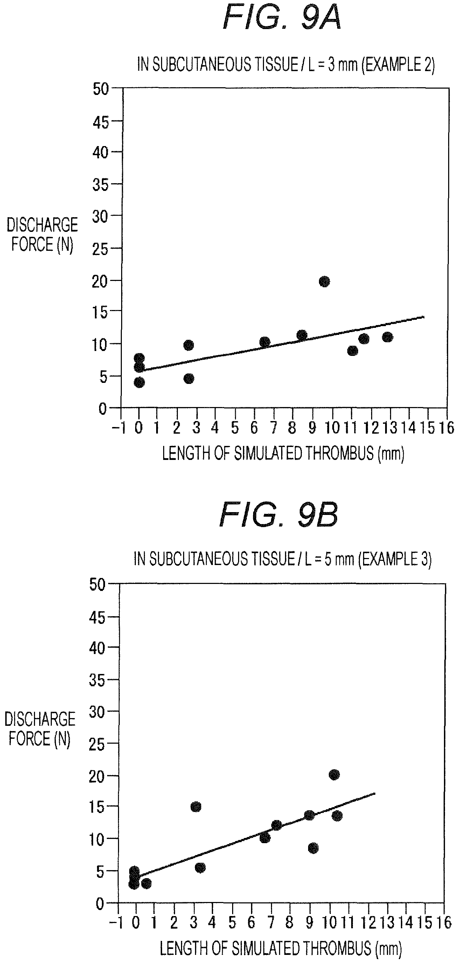

Fig. 9A is a graph illustrating a measurement result of a length of the simulated thrombus and an administration pressure in Example 2, and Fig. 9B is a graph illustrating a measurement result of a length of the simulated thrombus and an administration pressure in Example 3.

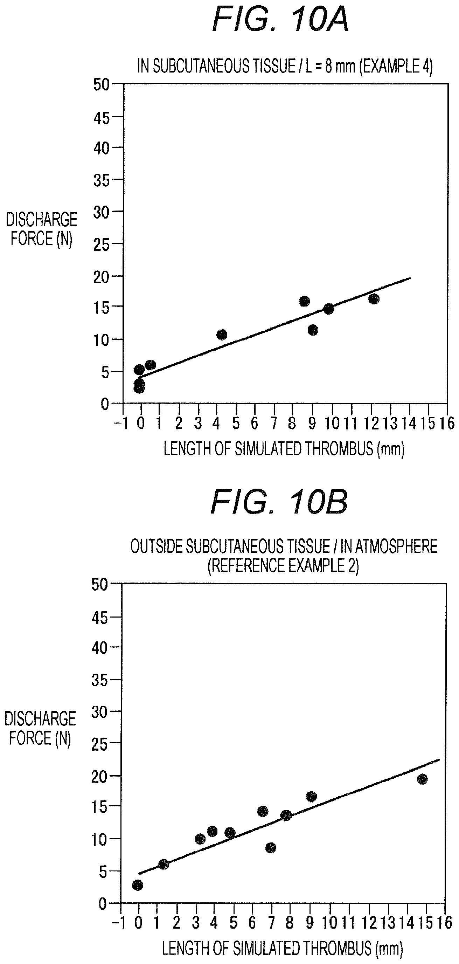

Fig. 10A is a graph illustrating a measurement result of a length of the simulated thrombus and an administration pressure in Example 4, and Fig. 10B is a graph illustrating a measurement result of a length of the simulated thrombus and an administration pressure in Reference Example 2 (in atmosphere).

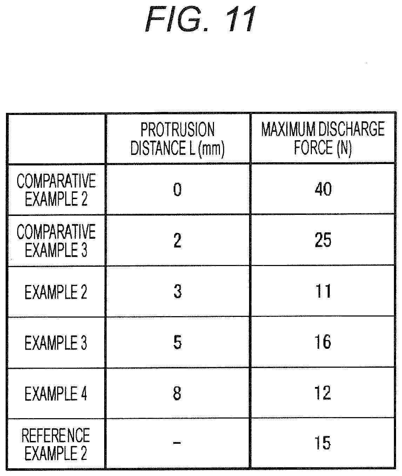

Fig. 11 is a table illustrating maximum discharge forces of Comparative Examples 2 and 3, Examples 2 to 4, and Reference Example 2 with respect to the simulated thrombus of a length of 10 mm or less.

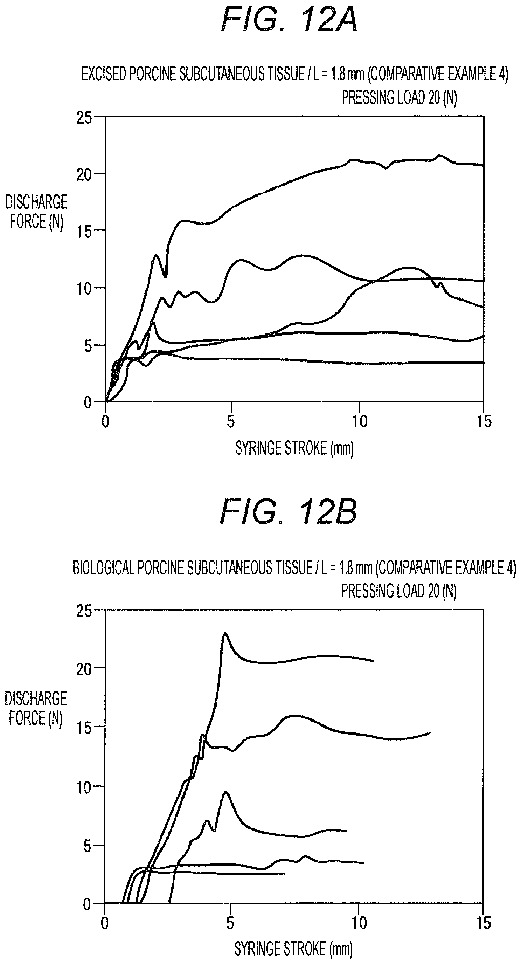

Fig. 12A is a graph illustrating transition of a discharge force of an indwelling needle for subcutaneous administration according to Comparative Example 4 in excised porcine subcutaneous tissue with a stroke distance of a syringe plotted along the abscissa and the discharge force plotted along the ordinate, and Fig. 12B is a graph illustrating transition of a discharge force of an indwelling needle for subcutaneous administration according to Comparative Example 4 in biological porcine subcutaneous tissue.

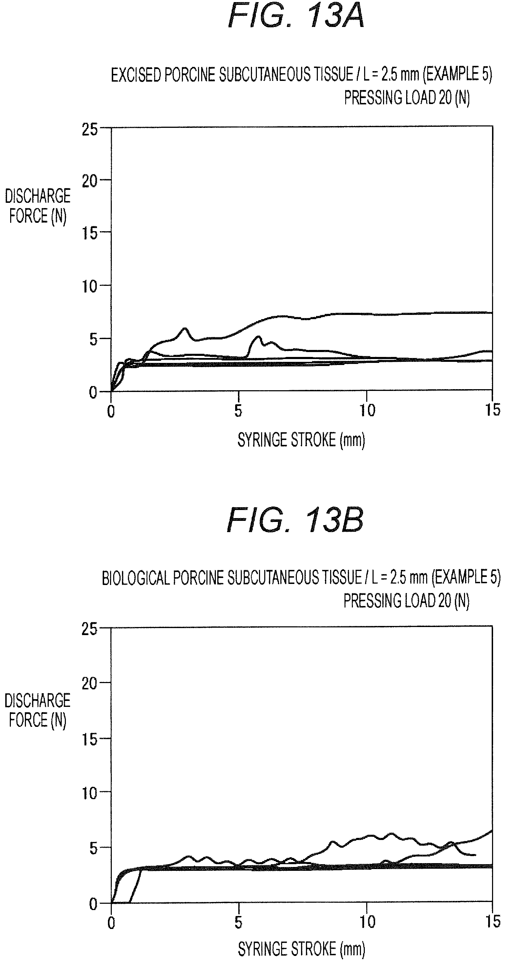

Fig. 13A is a graph illustrating transition of a discharge force of an indwelling needle for subcutaneous administration according to Example 5 in excised porcine subcutaneous tissue with a stroke distance of a syringe plotted along the abscissa and the discharge force plotted along the ordinate, and Fig. 13B is a graph illustrating transition of a discharge force of an indwelling needle for subcutaneous administration according to Example 5 in biological porcine subcutaneous tissue.

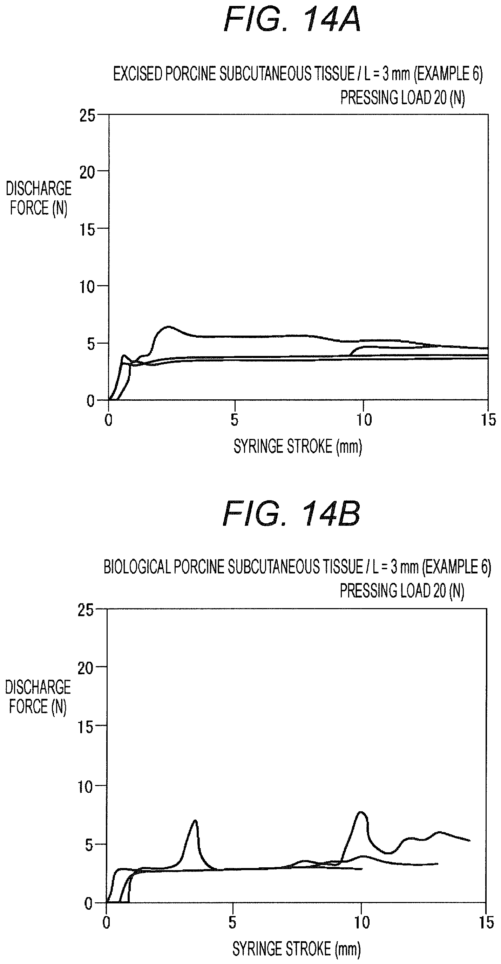

Fig. 14A is a graph illustrating transition of a discharge force of an indwelling needle for subcutaneous administration according to Example 6 in excised porcine subcutaneous tissue with a stroke distance of a syringe plotted along the abscissa and the discharge force plotted along the ordinate, and Fig. 14B is a graph illustrating transition of a discharge force of an indwelling needle for subcutaneous administration according to Example 6 in biological porcine subcutaneous tissue.

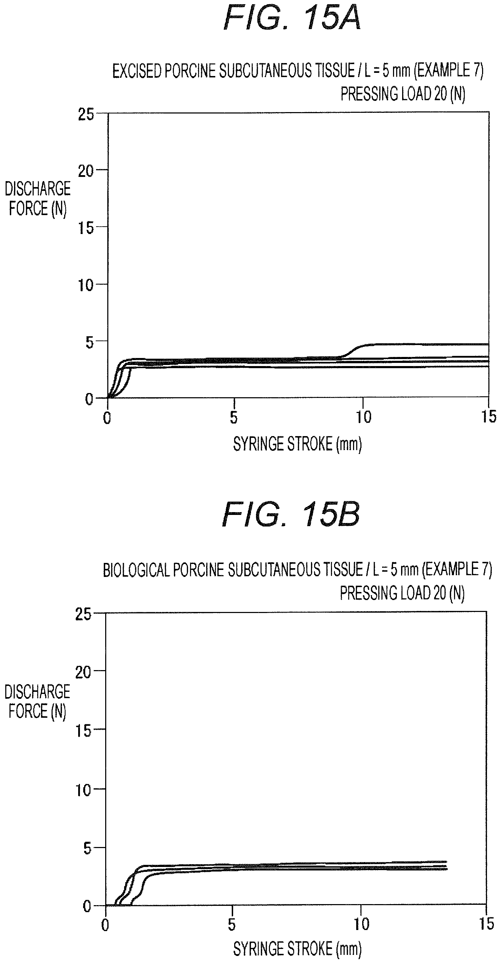

Fig. 15A is a graph illustrating transition of a discharge force of an indwelling needle for subcutaneous administration according to Example 7 in excised porcine subcutaneous tissue with a stroke distance of a syringe plotted along the abscissa and the discharge force plotted along the ordinate, and Fig. 15B is a graph illustrating transition of a discharge force of an indwelling needle for subcutaneous administration according to Example 7 in biological porcine subcutaneous tissue.

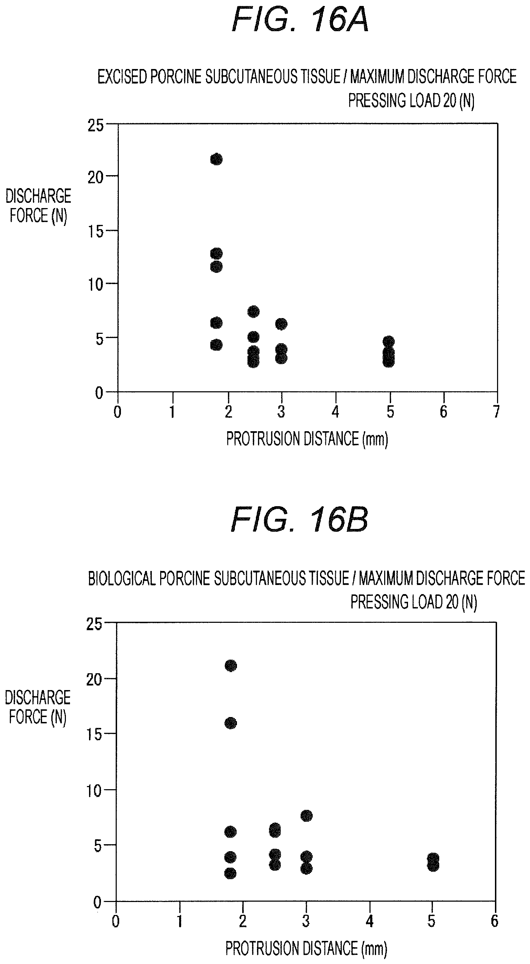

Figs. 16A and 16B are charts illustrating maximum discharge force distributions of Comparative Example 4 and Examples 5, 6, and 7.

### Description of Embodiments

Hereinafter, a preferred embodiment of an indwelling needle for subcutaneous administration is described in detail with reference to the accompanying drawings.

As illustrated in Fig. 1, an indwelling needle for subcutaneous administration 10 according to this embodiment is provided with an inner needle 12 and an outer needle 22 (catheter). As illustrated in the drawing, the indwelling needle for subcutaneous administration 10 punctures subcutaneous tissue of a living body in a state in which the inner needle 12 is mounted on the outer needle 22. After the indwelling needle for subcutaneous administration 10 punctures the subcutaneous tissue, the inner needle 12 is pulled out of the same, a medicinal solution administration device such as a syringe is mounted on the outer needle 22, and the indwelling needle for subcutaneous administration 10 is used for subcutaneously administering a medicinal solution to the living body.

The outer needle 22 (catheter) is provided with a tubular outer needle main body 18 and a hollow tubular outer needle hub 20 connected to a proximal end of the outer needle main body 18. The inner needle 12 includes a wire-shaped inner needle main body 14 having a circular cross-section and a cylindrical inner needle hub 16 connected to a proximal end of the inner needle main body 14. The inner needle 12 is inserted into a hub insertion portion 32 inside the outer needle 22, and in this state, a blade surface 24 formed at a distal end of the inner needle main body 14 protrudes from a distal end of the outer needle main body 18.

The outer needle main body 18 made of a resin material and the like, for example, is flexible and has appropriate elasticity, and is formed to be elongated in an axis G direction. In a state in which the inner needle 12 is mounted on the outer needle 22, the outer needle main body 18 reaches the vicinity of the distal end of the inner needle main body 14, and when the distal end of the inner needle main body 14 is inserted into subcutaneous tissue 90 by a predetermined length or more, a distal end 28 of the outer needle main body 18 is also inserted into the subcutaneous tissue 90 in the vicinity of the inner needle main body 14. Examples of a component of the outer needle main body 18 include various flexible resins such as ethylene-tetrafluoroethylene copolymer (ETFE), PTFE, polyurethane, and polyether nylon resin, for example.

The outer needle hub 20 is a resin member formed into a hollow tubular shape with both ends in the axis G direction opened. Examples of a component of the outer needle hub 20 include polyolefins such as polyethylene, polypropylene, and an ethylene-vinyl acetate copolymer, polyvinyl chloride, polymethyl methacrylate, polycarbonate, polyamide, polyester and the like, for example.

The proximal end of the outer needle main body 18 is connected and fixed to a distal end side of the outer needle hub 20. In the outer needle hub 20, the hub insertion portion 32 including a cylindrical inner surface into which the inner needle hub 16 is inserted is provided on a side closer to a proximal end than a connection portion to the outer needle main body 18. An inner diameter of the hub insertion portion 32 increases continuously or stepwise from a distal end toward a proximal end. A fitting surface substantially parallel to the axis G direction is formed in the hub insertion portion 32.

As illustrated in Fig. 2, the blade surface 24 inclined with respect to the axis G direction is formed at the distal end of the inner needle main body 14. As a component of the inner needle main body 14, a material of which a blade edge may be sharply formed to such an extent that sufficient puncture force (penetration force) may be obtained, the material having enough strength for puncture is used. Specific examples thereof include stainless steel, an aluminum alloy, a copper alloy and the like, for example. The inner needle main body 14 may be appropriately selected according to a size of the outer needle main body 18. For example, in a case where the outer needle main body 18 is of 14 G (outer diameter of 2.11 mm and inner diameter of 1.69 mm), the inner needle main body 14 may be of 16 G (outer diameter of 1.65 mm). The outer needle main body 18 may be of about 27 G to 14 G (outer diameter of 0.41 to 2.11 mm), and accordingly, the inner needle main body 14 may be of about 30 G to 16 G (outer diameter of 0.12 to 1.65 mm). A sharp needle tip 26 is formed at a distal end of the blade surface 24.

The needle tip 26 of the inner needle main body 14 protrudes by a predetermined protrusion distance L from the distal end 28 of the outer needle 22 in a state in which the inner needle 12 is mounted on the outer needle 22. The protrusion distance L is set to a length capable of forming a space of a sufficient length on a distal end side of the outer needle 22 in the subcutaneous tissue 90 after the inner needle 12 is pulled out. For example, the protrusion distance L is preferably set to 2.5 mm or longer. When the protrusion distance L is too long, a muscular layer at the back of the subcutaneous tissue 90 is punctured and the like, and invasion increases, so that the protrusion distance L is preferably set to 9 mm or shorter.

That is, the living body includes epidermis, dermis, the subcutaneous tissue, and the muscular layer in this order from a surface thereof, and the inner needle main body 14 of this embodiment punctures the dermis and the subcutaneous tissue to be used. Patients to whom the indwelling needle for subcutaneous administration 10 of this embodiment is assumed to be used are, for example, children around 12 years old to adults, and thicknesses of the dermis and the subcutaneous tissue vary depending on age and sex. The literature "Derraik, et al. PLOS ONE 2014, 9(1)" reports that the thicknesses of the dermis and the subcutaneous tissue are thinner in males than in females, and further thinner in children. According to this literature, an average value of the thickness of the dermis in an abdomen in males of 12 (±3.1) years old is 1.89 mm, and an average value of the thickness of the subcutaneous tissue is 9.13 mm.

Therefore, in the indwelling needle for subcutaneous administration 10 of this embodiment, the protrusion distance L of the inner needle main body 14 is set to 9 mm or shorter so as not to pierce the subcutaneous tissue even in a case where this punctures skin perpendicularly. Actually, the inner needle main body 14 and the outer needle main body 18 puncture the skin at an angle of 20° to 45°. For example, in a case where the abdomen is punctured at an angle of 20°, a length of about 32 mm is required even for children to puncture the muscular layer. Since the protrusion distance L of the inner needle main body 14 of this embodiment is sufficiently shorter than this value, it is possible to use with a margin. In a case of oblique puncture, the indwelling needle for subcutaneous administration 10 may also be used in a site other than the abdomen. The indwelling needle for subcutaneous administration 10 of this embodiment may also perpendicularly puncture the skin to be used.

Note that the protrusion distance L between the needle tip 26 and the distal end 28 of the outer needle 22 is the sum of a length in the axis direction of the blade surface 24 and a distance α in the axis direction between a heel 24a of the blade surface 24 and the distal end 28 of the outer needle main body 18. The length in the axis direction of the blade surface 24 is determined by a diameter of the inner needle main body 14 and may be set to about 1 mm. In this case, the distance α between the heel 24a of the blade surface 24 and the distal end 28 of the outer needle main body 18 may be set to 2 mm in order to set the protrusion distance L to 3 mm or longer.

As illustrated in Fig. 1, the proximal end of the inner needle main body 14 is connected to and held by a distal end of the inner needle hub 16. The inner needle hub 16 is a resin member formed into a hollow tubular shape with both ends in the axis G direction opened. Examples of a component of the inner needle hub 16 include the component similar to that of the outer needle hub 20 described above.

The proximal end of the inner needle main body 14 is connected and fixed to the distal end of the inner needle hub 16. At a distal end 38 of the inner needle hub 16 inserted into the hub insertion portion 32 of the outer needle hub 20, an abutment surface 36 extending substantially in parallel with the axis G direction that abuts the fitting surface when the inner needle 12 is mounted on the outer needle 22 is formed. The inner needle 12 and the outer needle 22 are configured to be fixed to each other by a frictional force of a fitting portion.

The indwelling needle for subcutaneous administration 10 according to this embodiment is basically configured as described above, and an action and an effect thereof are hereinafter described.

First, the inner needle 12 is mounted on the outer needle 22. The inner needle 12 is mounted in such a manner that the inner needle main body 14 is inserted from the proximal end side of the outer needle hub 20. The inner needle main body 14 is inserted along a lumen 30 of the outer needle main body 18 via the outer needle hub 20, and finally the inner needle hub 16 is inserted and pushed into the outer needle hub 20 to abut each other, so that the inner needle hub 16 stops and the inner needle 12 and the outer needle 22 are connected to each other. As a result, the indwelling needle for subcutaneous administration 10 is assembled in a state in which the needle tip 26 of the inner needle main body 14 protrudes from the distal end 28 of the outer needle main body 18 by the protrusion distance L.

At the time of puncture with the indwelling needle for subcutaneous administration 10, first, a medical worker such as a doctor or the patient himself/herself grasps the indwelling needle for subcutaneous administration 10 and punctures the subcutaneous tissue 90 of the patient with the same, and gradually inserts the indwelling needle for subcutaneous administration 10 toward a desired site. As a result, as illustrated in Fig. 3A, the indwelling needle for subcutaneous administration 10 advances while the needle tip 26 of the indwelling needle for subcutaneous administration 10 cuts the subcutaneous tissue 90 open.

Thereafter, as illustrated in Fig. 3B, the inner needle 12 is removed from the outer needle 22. According to the indwelling needle for subcutaneous administration 10 of this embodiment, the inner needle main body 14 of the inner needle 12 protrudes from the outer needle 22 by a predetermined length L. As a result, a gap 92 (tear) of a sufficient length remains in the subcutaneous tissue 90 on a distal end side of the distal end 28 of the outer needle 22 by removal of the inner needle 12.

Thereafter, a portion of the outer needle 22 exposed from the skin of the patient is fixed to the skin of the patient, and the syringe filled with a medicinal solution is connected to the outer needle hub 20 to perform subcutaneous administration of the medicinal solution. As illustrated in Fig. 4A, there is a case where displacement when the outer needle 22 is fixed or displacement when the syringe is connected is transmitted to the distal end 28 of the outer needle main body 18, and the distal end 28 of the outer needle main body 18 is pushed to the distal end side. In contrast, in the indwelling needle for subcutaneous administration 10 of this embodiment, the gap 92 of the sufficient length is formed on a distal end side of the outer needle main body 18, so that the displacement of the outer needle main body 18 may be absorbed by the gap 92. As a result, breakage (kink) of the outer needle main body 18 may be prevented. Since the gap 92 is formed on the distal end of the outer needle main body 18, the lumen 30 of the outer needle 22 is less likely to be blocked by the subcutaneous tissue 90, so that the medicinal solution may be reliably injected.

When the outer needle 22 is indwelled in the subcutaneous tissue 90, as illustrated in Fig. 4B, blood, protein and the like in the living body might enter the lumen 30 of the outer needle main body 18 due to a biological reaction and they might generate a thrombotic precipitate 94. Even in such a case, according to the indwelling needle for subcutaneous administration 10 of this embodiment, the gap 92 of the sufficient length is formed on the distal end side of the outer needle main body 18, so that the precipitate 94 may be smoothly discharged into the gap 92. As a result, the precipitate 94 may be discharged without increasing an injection pressure of the medicinal solution, and the medicinal solution may be reliably administered subcutaneously to the living body.

Hereinafter, results of experiments performed to confirm the effect of the indwelling needle for subcutaneous administration 10 of this embodiment are described.

### (Experimental Example 1)

In Reference Example 1, an outer needle 22 (thickness of 24 G) of an indwelling needle for subcutaneous administration 10 was prepared, and in a lumen 30 in the vicinity of a distal end 28 thereof, a simulated thrombus 98 of a length of about 11 mm in an axis direction was formed. Thereafter, a syringe containing physiological saline was connected to an outer needle hub 20 of the outer needle 22, and a pressure required for discharging the simulated thrombus 98 from the outer needle 22 was measured. The measurement in Reference Example 1 was performed five times.

In Example 1, the simulated thrombus 98 of a length of about 11 mm in the axis direction was arranged in the lumen 30 of the outer needle 22 of the indwelling needle for subcutaneous administration 10 in Fig. 1, and after indwelling this in porcine subcutaneous tissue 100, an inner needle 12 was pulled out to form a gap 92 as illustrated in Fig. 5. Note that a protrusion distance L of a needle tip 26 of the inner needle 12 from the outer needle 22 was set to 4 mm. The outer needle 22 of a thickness of 24 G was used.

Thereafter, the physiological saline was injected from the syringe connected to the outer needle hub 20, and a pressure (input load to the syringe) at which the simulated thrombus 98 was discharged from the outer needle 22 was measured. The measurement in Example 1 as described above was performed five times.

In Comparative Example 1, using the outer needle 22 similar to that in Example 1, the outer needle 22 was indwelled in the porcine subcutaneous tissue 100 as illustrated in Fig. 5, and a pressure required for discharging the simulated thrombus 98 was measured. Note that, in Comparative Example 1, the protrusion distance L of the inner needle 12 when puncturing the subcutaneous tissue 100 with the outer needle 22 was set to 0 mm. That is, in Comparative Example 1, the pressure (input load to the syringe) required for discharging the simulated thrombus 98 was measured under a condition that the gap 92 was not formed on a distal end side of the outer needle 22. In Comparative Example 1 also, the measurement was performed five times.

As illustrated in a result of Reference Example 1 in Fig. 6A, a pressure of liquid supplied from the syringe increases with an increase in stroke of the syringe, and the simulated thrombus 98 is discharged from the outer needle 22 at a predetermined pressure, then the pressure decreases. The pressure required for discharging the simulated thrombus 98 is obtained as a maximum pressure immediately before the pressure decreases with respect to the increase in stroke. As illustrated in the drawing, it may be found that the simulated thrombus 98 is discharged from the outer needle 22 with the input load of about 10 N at the maximum in Reference Example 1.

As illustrated in a result of Example 1 in Fig. 6B, it was confirmed that the simulated thrombus 98 was discharged from the outer needle 22 with the input load of about 12 N at the maximum in a case where the needle tip 26 of the inner needle 12 was protruded by 4 mm. This value is close to the result of Reference Example 1 outside the skin, and it may be found that the simulated thrombus 98 may be discharged at the pressure equivalent to that outside the skin according to Example 1 of this embodiment.

In contrast, in a result of Comparative Example 1 in Fig. 7, there was a case where the input load close to 20 N was required for discharging the simulated thrombus 98, resulting in an increase in discharge pressure of a medicinal solution at the time of subcutaneous administration.

As described above, it was confirmed that the thrombus may be discharged from the outer needle 22 without increasing the discharge pressure, and the subcutaneous administration of the medicinal solution may be reliably performed.

### (Experimental Example 2)

In Experimental Example 2, a discharge force for a simulated thrombus 98 was evaluated. That is, the simulated thrombus 98 made of gelatin of various lengths was arranged in an outer needle 22 of an indwelling needle for subcutaneous administration 10, this was indwelled in porcine subcutaneous tissue 100 that is excised (excised porcine subcutaneous tissue), and the discharge force for this was measured by an autograph (automatic load measurement device). The outer needle 22 of a thickness of 24 G was used. A protrusion distance L of an inner needle 12 from the outer needle 22 was set to 0 mm (Comparative Example 2), 2 mm (Comparative Example 3), 3 mm (Example 2), 5 mm (Example 3), and 8 mm (Example 4). The discharge force when the outer needle 22 did not puncture the subcutaneous tissue 100 and the simulated thrombus 98 was discharged in the atmosphere was measured as Reference Example 2.

As illustrated in Figs. 8A and 8B, from results of Comparative Example 2 (L = 0 mm) and Comparative Example 3 (L = 2 mm), it is found that the discharge force for the simulated thrombus 98 significantly varies and there is a case where a large discharge force is required. Focusing on a practically important range in which the length of the simulated thrombus 98 is 10 mm or shorter, as illustrated in Fig. 11, a large discharge force of 40 N or larger at the maximum is required in Comparative Example 2, and a large discharge force of about 25 N at the maximum might be required in Comparative Example 3. In this manner, in Comparative Examples 2 and 3, a large administration pressure might be generated when discharging the simulated thrombus 98.

In contrast, as illustrated in Figs. 9A to 10A, it is found that the variation in discharge force is reduced and an injection pressure is stabilized in results of Examples 2, 3, and 4. Focusing on a range in which the length of the simulated thrombus 98 is 10 mm or less, as illustrated in Fig. 11, the maximum value of the discharge force is about 11 N in Example 2 (L = 3 mm), about 16 N in Example 3 (L = 5 mm), and about 12 N in Example 4 (L = 8 mm), and it is found that the maximum value of the discharge force is suppressed as compared with that in Comparative Examples 2 and 3. The results of Examples 2, 3, and 4 are equivalent to 15 N, which is the maximum value of the discharge force in Reference Example 2 to discharge the simulated thrombus 98 in the atmosphere illustrated in Fig. 10B, and it is found that the protrusion distance L of the inner needle 12 is effectively set to 3 mm or longer when discharging the simulated thrombus 98.

### (Experimental Example 3)

In a case of indwelling an indwelling needle for subcutaneous administration 10 in subcutaneous tissue of a patient to use, it is assumed that a pressing load acts on an outer needle 22 of the indwelling needle for subcutaneous administration 10 as the patient rolls over and the like. When such pressing load acts, there is a possibility that a position of a distal end of the outer needle 22 changes and an administration pressure changes. Therefore, in this experimental example, the administration pressure (discharge force) in a case where the pressing load is applied to the outer needle 22 indwelled in the subcutaneous tissue was evaluated.

In this experimental example, the outer needle 22 was indwelled in excised porcine subcutaneous tissue and biological porcine subcutaneous tissue, and while applying a pressing load of 20 N to the outer needle 22 and the skin around the same with a push-pull gauge, the discharge force (administration pressure) of physiological saline was measured by an autograph. A thickness of the outer needle 22 was of 24 G, and an insertion angle of the outer needle 22 was set to 20°. Here, the pressing load of 20 N is assumed to be a load applied to the outer needle 22 indwelled in the abdomen when an adult of a body weight of 60 kg lies down.

In this experimental example, measurement was performed for Comparative Example 4 in which a protrusion distance L of an inner needle 12 when puncturing the subcutaneous tissue with the indwelling needle for subcutaneous administration 10 was set to 1.8 mm, Example 5 in which the protrusion distance L was set to 2.5 mm, Example 6 in which the protrusion distance L was set to 3 mm, and Example 7 in which the protrusion distance L was set to 5 mm. In this experimental example, the measurement was performed five times for the excised porcine subcutaneous tissue and the biological porcine subcutaneous tissue, respectively.

As illustrated in Figs. 12A and 12B, it was found that variation in discharge force was large in both the excised porcine subcutaneous tissue and the biological porcine subcutaneous tissue, and a maximum discharge force might become a large value of 20 N or more in Comparative Example 4 (L = 1.8 mm).

As illustrated in Figs. 13A and 13B, it is found that a range of variation in discharge force is smaller than that in Comparative Example 4 and the discharge force is smaller for both the excised porcine subcutaneous tissue and the biological porcine subcutaneous tissue in Example 5 (L = 2.5 mm).

As illustrated in Figs. 14A and 14B, it is found that a range of variation in discharge force is smaller than that in Comparative Example 4 and the discharge force is smaller for both the excised porcine subcutaneous tissue and the biological porcine subcutaneous tissue in Example 6 (L = 3 mm) .

Furthermore, as illustrated in Figs. 15A and 15B, it is found that a range of variation in discharge force is smaller than that in Comparative Example 4 and the discharge force is smaller for both the excised porcine subcutaneous tissue and the biological porcine subcutaneous tissue in Example 7 (L = 5 mm).

As illustrated in Figs. 16A and 16B, focusing on the maximum discharge force, in Comparative Example 4, the maximum discharge force is a large value such as 21 N for the excised porcine subcutaneous tissue and 24 N for the biological porcine subcutaneous tissue. In contrast, it is found that the maximum discharge force in Example 5 is a small value such as 7 N in the excised porcine subcutaneous tissue and 6 N in the biological porcine subcutaneous tissue, and the maximum discharge force in Example 6 is a small value such as 6 N in the excised porcine subcutaneous tissue and 8 N in the biological porcine subcutaneous tissue. It was confirmed that the maximum discharge force in Example 7 was 5 N in the excised porcine subcutaneous tissue and 4 N in the biological porcine subcutaneous tissue, which was smaller than that in Examples 5 and 6.

The variation in maximum discharge force in Examples 5, 6, and 7 is smaller than that in Comparative Example 4. From this result, it is found that the protrusion distance L of the inner needle 12 of the indwelling needle for subcutaneous administration 10 is effectively set to 2.5 mm or longer for stabilizing the administration pressure under an environment in which the load acts.

The indwelling needle for subcutaneous administration 10 of this embodiment has the following effects.

The indwelling needle for subcutaneous administration 10 is provided with an inner needle 12 including an inner needle main body 14 at a distal end of which a sharp needle tip 26 is formed, and an inner needle hub 16 provided on a proximal end side of the inner needle main body 14, and an outer needle 22 including an outer needle main body 18 including a lumen 30 through which the inner needle 12 is inserted, and an outer needle hub 20 provided on a proximal end side of the outer needle main body 18 in which a hub insertion portion 32 in which the inner needle hub 16 is mounted is formed, in which the needle tip 26 of the inner needle 12 protrudes by 2.5 to 9 mm in an axis direction from a distal end 28 of the outer needle 22 in a state in which the inner needle hub 16 is mounted in the outer needle hub 20. As a result, it is possible to form the gap 92 of the sufficient length on the distal end side of the outer needle 22 in the subcutaneous tissue 90 after the inner needle 12 is pulled out. By forming such gap 92, it is possible to prevent the breakage (kink) or blockage of the outer needle 22 and reliably administer the medicinal solution.

In the indwelling needle for subcutaneous administration 10, the protrusion distance L of the needle tip 26 of the inner needle 12 from the distal end 28 of the outer needle 22 may be set to 5 mm or shorter. With this configuration, it is possible to prevent damage on an unnecessary portion by the needle tip 26.

The indwelling needle for subcutaneous administration 10 may be configured such that the inner needle 12 includes the blade surface 24 inclined with respect to the axis direction, and the heel 24a at the proximal end of the blade surface 24 protrudes to the distal end side from the outer needle 22. This makes it possible to secure a desired protrusion distance L without changing the angle of the blade surface 24. In this case, the heel 24a may be configured to protrude to the distal end side by 1 mm or more from the distal end 28 of the outer needle 22. Since the indwelling needle for subcutaneous administration 10 is intended to perform subcutaneous puncture, it is not necessary to take damage to a blood vessel into consideration, and there is no problem even if the heel 24a protrudes to the distal end side by 1 mm or more from the distal end 28 of the outer needle 22.

In the indwelling needle for subcutaneous administration 10, the length in the axial direction of the blade surface 24 may be set to 1 mm or more in place of the protrusion distance L of the heel 24a or in addition to adjustment of the protrusion distance L of the heel 24a. The protrusion distance L may be adjusted not only by a position of the heel 24a but also by the length in the axis direction of the blade surface 24.

The indwelling needle for subcutaneous administration is described above with reference to the preferred embodiment, but the indwelling needle for subcutaneous administration is not limited to the above-described embodiment, and it goes without saying that various modifications may be made without departing from the gist of the present invention.

## Claims

1. An indwelling needle for subcutaneous administration indwelled in subcutaneous tissue for subcutaneously administering a medicinal solution, the indwelling needle for subcutaneous administration comprising:
an inner needle including an inner needle main body at a distal end of which a sharp needle tip is formed, and an inner needle hub provided on a proximal end side of the inner needle main body; and
an outer needle including an outer needle main body including a lumen through which the inner needle is inserted, and an outer needle hub provided on a proximal end side of the outer needle main body in which a hub insertion portion in which the inner needle hub is mounted is formed, wherein
the needle tip of the inner needle protrudes by 2.5 to 9 mm in an axis direction from a distal end of the outer needle in a state in which the inner needle hub is mounted in the outer needle hub.

2. The indwelling needle for subcutaneous administration according to claim 1, wherein
a protrusion distance of the needle tip of the inner needle from the distal end of the outer needle is 5 mm or shorter.

3. The indwelling needle for subcutaneous administration according to claim 1 or 2, wherein
the inner needle includes a blade surface inclined with respect to the axis direction, and a heel at a proximal end of the blade surface protrudes to a distal end side from the outer needle.

4. The indwelling needle for subcutaneous administration according to claim 3, wherein
the heel protrudes to the distal end side by 1 mm or more from the distal end of the outer needle.

5. The indwelling needle for subcutaneous administration according to claim 3 or 4, wherein
a length in the axis direction of the blade surface is 1 mm or more.
